# EUROPEAN PATENT APPLICATION

(11) **EP 4 520 321 A2**
(43) Date of publication of application: **12.03.2025**
(21) Application number: 24204396.6
(22) Date of filing: 03.05.2021
(51) Int. Cl.: A61K 9/127, A61K 39/395, A61K 41/00, A61K 45/06, A61P 35/00, C07K 16/00

(54) **COMPOSITIONS COMPRISING ANTI-VEGF AND NANOPARTICLES AND METHODS OF USING THE SAME FOR THE TREATMENT OF ABNORMAL OR EXCESSIVE ANGIOGENESIS**

(30) Priority: 01.05.2020 US 202063018786 P
(62) Divisional of application: 21727703.7
(71) Applicant: Pham, Randal Tanh Hoang, Los Gatos, CA 95032 (US); Bernstein, Eric F., Gladwyne PA 19003 (US); Koop, Dale, Woodside CA 94062 (US)
(72) Inventor: Pham, Randal Tanh Hoang, Los Gatos, CA 95032 (US); Bernstein, Eric F., Gladwyne PA 19003 (US); Koop, Dale, Woodside CA 94062 (US)
(74) Representative: HGF

(57) **Abstract**

A composition for treating abnormal or excessive angiogenesis, such as pyogenic granuloma comprising an anti-vascular endothelial growth factor (anti-VEGF) agent (*e.g.,* an antibody or small molecule inhibitor of VEGF signaling) and a carrier comprising nanoparticles. Methods of treating abnormal or excessive angiogenesis by administering a composition comprising an anti-VEGF agent and nanoparticles, alone or in combination with administering an anti-inflammatory steroid, and administering a non-steroidal anti-inflammatory drug (NSAID) to a subject. Devices for administering the composition for treating pyogenic granuloma are also disclosed.

## Description

### Priority Data

This application claims the benefit of priority to Provisional Application No. 63/018,786, filed May 1, 2020, the entire contents of which are expressly incorporated herein by reference.

### Technical Field

The present disclosure relates generally to compositions comprising anti-VEGF and a nanoparticle delivery system for treating disorders of the eye and/or skin, including pyogenic granuloma. The present disclosure also relates to methods of treating such eye and/or skin conditions by applying the described composition to the affected area of the skin or the periorbital and/or orbit area of an eye.

### Background

Anti-vascular endothelial growth factor therapy, also known as anti-VEGF therapy or anti-VEGF medication, is the use of medications that block vascular endothelial growth factor, including but not limited to vascular endothelial growth factor receptors 1, 2 and 3. Traditionally, this method has been used in the treatment of certain cancers and in age-related macular degeneration.

Anti-VEGF drugs have shown therapeutic efficacy in mouse models of cancer and in an increasing number of human cancers. Such drugs can include monoclonal antibodies such as bevacizumab (sold as Avastin^{®}), antibody derivatives such as ranibizumab (sold as Lucentis^{®}), or orally available small molecules that inhibit the tyrosine kinases stimulated by VEGF.

Off-label use of intravitreal bevacizumab has become a widespread treatment for neovascular age-related macular degeneration (AMD). Some studies suggest that bevacizumab is effective in increasing visual acuity with low rates of ocular adverse effects.

Bevacizumab is a 149-kD humanized monoclonal antibody that inhibits vascular endothelial growth factor (VEGF), a signal protein that stimulates angiogenesis and vasculogenesis in age-related macular degeneration (AMD). While bevacizumab received FDA approval for use in the management of various cancers, compounded bevacizumab has been used off label in the treatment of ophthalmic conditions including AMD since May 2005. Today, bevacizumab is successfully used to treat a variety of conditions, including diabetic retinopathy, central retinal vein occlusion, neovascular glaucoma, and retinopathy of prematurity, in addition to a host of other less common eye diseases.

There is a need for a composition for the treatment of abnormal or excessive angiogenesis, such as ocular pyogenic granuloma manifestation after pterygium removal surgery, as described in Example 1. The disclosed compositions comprising anti-VEGF and a nanoparticle delivery system are directed to overcoming one or more of the problems set forth above and/or other problems of the prior art.

### Summary

In one aspect, the present disclosure is directed to a composition for the treatment of abnormal or excessive angiogenesis. The composition comprises at least one anti-vascular endothelial grown factor (anti-VEGF) antibody; and a carrier comprising nanoparticles, wherein the nanoparticles are organic, inorganic, or a combination thereof.

In another aspect, the present disclosure is directed to a method of treating abnormal or excessive angiogenesis. In one embodiment, the method comprises administering to an area containing abnormal or excessive angiogenesis a composition containing at least one anti-VEGF antibody and nanoparticles, wherein the nanoparticles are organic, inorganic, or a combination thereof. The method may further comprise administering additional pharmaceutical compounds, such as one or more anti-inflammatory steroids and one or more non-steroidal, anti-inflammatory drug (NSAID).

In another aspect, the present disclosure is directed to a device for administering the disclosed composition. In one embodiment, the device is configured to administer the composition by at least one method selected from intravenous, subconjunctival, subtenon, episcleral, intrascleral, subscleral, intraperitoneal, epidural, intrathecal, intramuscular, intraluminal, intratracheal, epidermal, intradermal, subdermal or subcutaneous.

### Brief Description of the Drawings

The accompanying figures are incorporated in and constitute a part of this specification.
**FIG. 1****.** is a pre-operation picture of a patient's eye as described in Example 1.
**FIG. 2** is a post-operation picture of a patient's eye showing a pyogenic granuloma lesion as described in Example 1.
**FIG. 3** is a picture of a patient's eye after the injection of an anti-inflammatory steroid (Kenalog) and before the injection of an anti-VEGF agent, showing a reduced pyogenic granuloma lesion as described in Example 1.
**FIG. 4** is a picture of a patient's eye 18 days after the injection of both an anti-inflammatory steroid (Kenalog) and an anti-VEGF agent (bevacizumab), showing a significant reduction in the pyogenic granuloma lesion, as described in Example 1.

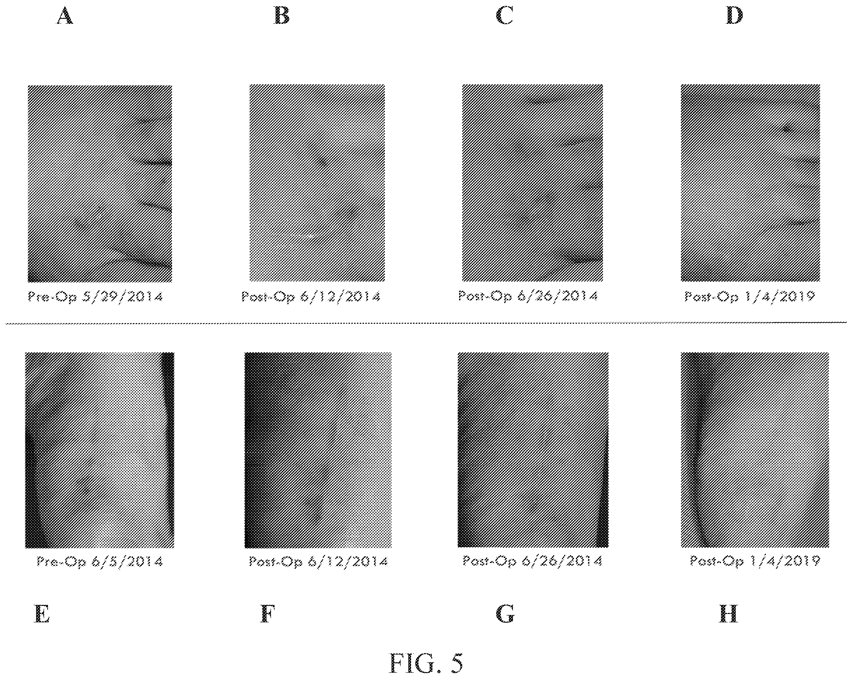

**FIGS. 5A, 5B, 5C**, **5D** are photographs showing the effect of using anti-VEGF according to the present disclosure use on hypertrophic scars from pre-operation (**FIG. 5A****)** and at various intervals after treatment with bevacizumab injection including two weeks post-op **(****FIG. 5B****),** four weeks post-op (**FIG. 5C****)** and about seven months post-op (**FIG. 5D****).**
**FIGS. 5E, 5F, 5G, 5H** are photographs showing the effect of using anti-VEGF according to the present disclosure use on hypertrophic scars from pre-operation **(****FIG. 5E****)** and at various intervals post-operation, including one week post-op **(****FIG. 5F****),** three weeks post-op **(****FIG. 5G****)** and about seven months post-op **(****FIG. 5H****).**

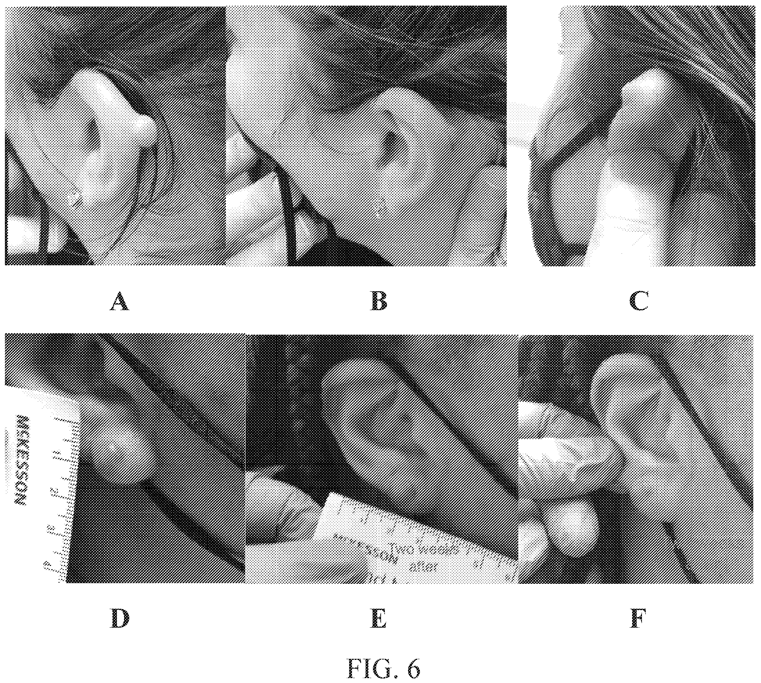

**Figs. 6A, 6B, 6C** are photographs showing a keloid on the left ear of a patient prior to laser surgery **(****FIG. 6A**); three weeks after laser surgery (**FIG. 6B****),** and two weeks after an intralesional bevacizumab injection (**FIG. 6C****).**
**Figs. 6D, 6E, 6F** are photographs showing a keloid on the right ear of a patient prior to treatment with intralesional bevacizumab injection **(****FIG. 6D****);** and two weeks after an intralesional bevacizumab injection (**FIG. 6E** and **6F****).**

### Detailed Description of Disclosure

### Definitions:

As used herein, the term "ocular pyogenic granuloma" is the manifestation of a pterygium in the eye after pterygium removal surgery.

As used herein, the term "subject" means any mammal, and, in particular, a human, and can also be referred to, e.g., as an individual or patient.

A "subject in need of treatment" for ocular pyogenic granuloma according to the methods disclosed herein is a subject who is at risk of ocular pyogenic granuloma, e.g., a patient who is about to undergo or has recently (e.g., within about 12, 11, 10, 9, 8, 7, 6, 5, 4, 3, 2 or 1 month(s), about 2 or 1 week(s) or less) undergone, or is about to undergo (e.g, within about 1 month, about 1 week or less) surgery to remove a pterygium, keloid, or scar.

As used herein, an "anti-VEGF agent" means an inhibitor of VEGF signaling. Anti-VEGF agents include antibodies (e.g., bevacizumab), antibody fragments (e.g., an antibody light chain (VL), an antibody heavy chain (VH), a single chain antibody (scFv), an F(ab')2 fragment, a Fab fragment, an Fd fragment, an Fv fragment, and a single domain antibody fragment (DAb). Fragments can be obtained, e.g., via chemical or enzymatic treatment of an intact or complete antibody or antibody chain or by recombinant means), fusion proteins, peptide, nucleic acids (e.g., siRNA, shRNA), and other small molecules, etc. that disrupt the interaction between VEGF (VEGF-A or VEGF-C/VEGF-D) and its receptor (VEGFR-1,/VEGFR-2 or VEGFR-3, respectively). Other, non-limiting examples of anti-VEGF agents encompassed by the present disclosure are provided herein below.

As used herein, the term "adjacent to", e.g., in the context of injecting an anti-VEGF adjacent to or near the site of new blood vessel growth, means proximate to (e.g., within about 0.1 mm, 0.2 mm, 0.3 mm, 0.4 mm, 0.5 mm, 1 mm, 2 mm, 3 mm, 4 mm, or 5 mm from the site of blood vessel growth).

As used herein the terms "therapeutically effective" and "effective amount", used interchangeably, applied to a dose or amount refer to a quantity of a composition, compound or pharmaceutical formulation that is sufficient to result in a desired activity upon administration to a subject in need thereof. Within the context of the present invention, the term "therapeutically effective" refers to that quantity of a composition, compound or pharmaceutical formulation that is sufficient to reduce, eliminate or delay at least one symptom of a disease or condition specified herein, e.g., pterygium or keloid recurrence. When a combination of active agents is administered, the effective amount of the combination, or individual agents, may or may not include amounts of each agent that would have been effective if administered individually. The dosage of the therapeutic formulation will vary, depending upon the nature of the disease or condition, the patient's medical history, the frequency of administration, the manner of administration, the clearance of the agent from the host, and the like. The initial dose may be larger, followed by smaller maintenance doses. The dose may be administered, e.g., weekly, biweekly, daily, semi-weekly, etc., to maintain an effective dosage level.

Therapeutically effective dosages in the methods described herein can be determined by the treating physician. For example, the physician may begin treatment using manufacturer-recommended doses for the anti-VEGF agent, anti-inflammatory steroid and/or NSAID, and adjust based on the physician's observations of the effect of treatment. Further guidance is provided herein and in the Examples. In addition, clinical trials can be conducted to determine the doses that are effective to produce statistically significant treatment effects when a population of patients is treated

As used herein "combination therapy" means the treatment of a subject in need of treatment with a certain composition or drug in which the subject is treated or given one or more other compositions or drugs for the disease in conjunction with the first and/or in conjunction with one or more other therapies, such as, eg., surgery. Such combination therapy can be sequential therapy wherein the patient is treated first with one treatment modality (e.g., drug or therapy), and then the other (e.g., drug or therapy), and so on, or all drugs and/or therapies can be administered simultaneously. In either case, these drugs and/or therapies are said to be "co-administered." It is to be understood that "co-administered" does not necessarily mean that the drugs and/or therapies are administered in a combined form (i.e., they may be administered separately or together to the same or different sites at the same or different times).

The phrase "pharmaceutically acceptable" is employed herein to refer to those compounds, materials, compositions and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "treating" or "treatment" of a state, disorder or condition (e.g., pterygium or keloid recurrence) includes: (1) preventing or delaying the appearance of clinical or sub-clinical symptoms of the state, disorder or condition developing in a mammal that may be afflicted with or predisposed to the state, disorder or condition but does not yet experience or display clinical or subclinical symptoms of the state, disorder or condition; and/or (2) inhibiting the state, disorder or condition, including arresting, reducing or delaying the development of the disease or a relapse thereof (in case of maintenance treatment) or at least one clinical or sub-clinical symptom thereof; and/or (3) relieving the disease, i.e., causing regression of the state, disorder or condition or at least one of its clinical or sub-clinical symptoms; and/or (4) causing a decrease in the severity of one or more symptoms of the disease. The benefit to a subject to be treated is either statistically significant or at least perceptible to the patient or to the physician.

### Description of Abnormal or Excessive Angiogenesis

The disclosed composition, devices, and methods can be used to treat abnormal or excessive angiogenesis forming on the skin or in the eye. Angiogenesis is a physiological process through which new blood vessels form pre-existing vessels by continuing the growth of the vasculature system by sprouting or splitting from existing vessels. Normal angiogenesis is a necessary process in the growth and development of the human body, including wound healing and the formation of granulation tissue. Angiogenesis is regulated by a very sensitive interplay of growth factors and inhibitors, and their imbalance can lead to disease. One of the growth factors are chemical stimulators such as VEGF. VEGF has been demonstrated to be a major contributor to angiogenesis, increasing the number of capillaries in a given network. An imbalance in this interplay may result in abnormal or excessive angiogenesis that can lead to other complicating health factors.

For example, abnormal or excessive angiogenesis can result in the excessive growth of the vasculature, resulting in many debilitating conditions such as cancer, skin diseases, age-related blindness, diabetic ulcers, cardiovascular disease, stroke, and many others. Specifically, overexpression of VEGF may cause increased permeability in blood vessels in addition to stimulating angiogenesis. In wet macular degeneration, VEGF causes proliferation of capillaries into the retina. Since the increase in angiogenesis also causes edema, blood and other retinal fluids leak into the retina, causing loss of vision.

In some embodiments, complications of abnormal or excessive angiogenesis can result in one or more of the following conditions: pyogenic granuloma, infantile hemangioma, hemangioma of infancy, congenital hemangioma, tufted hemangioma, spindle cell hemangioma, epithelioid hemangioma, kaposiform hemangioendothelioma, retiform hemangioendothelioma, papillary intralymphatic angioendothelioma, dabska tumor, composite hemangioendothelioma, Kaposi sarcoma, angiosarcoma, Epithelioid hemangioendothelioma, capillary malformation, lymphatic malformation, venous malformation, arteriovenous malformation, arteriovenous fistula, capillary-lymphatic malformation, capillary-arteriovenous malformation, capillary-lymphatic-arteriovenous malformation, capillary-venous-arteriovenous malformation, capillary-lymphatic-venous-arteriovenous malformation.

### Description of Anti-VEGF Agent

The human VEGF-A gene is organized in eight exons. Alternative exon splicing results in the generation of four main VEGF isoforms, having, respectively, 121, 165, 189, and 206 amino acids following signal sequence cleavage (VEGF121, VEGF165, VEGF189, and VEGF206). VEGF165 is believed to be the most physiologically relevant isoform. For a review, see Ferrara et al., Biochem. Biophys. Res. Commun., 2005, 333, 328-335. The amino acid sequences of VEGF-A are well known in the art, and due to splice variation, the sequences are numerous. By way of non-limiting example, the following are exemplary and non-limiting GenBank.RTM. Accession Nos. for human VEGF-A amino acid sequences: AAP86646.1, P15692.2, NP_001191313.1, NP_001165101.1, NP_001165099.1, NP_001165097.1, NP_001165095.1, NP_001020539.2, NP_003367.4, NP_001165093.1, NP_001020541.2, NP_001191314.1, NP_001165100.1, NP_001165098.1, NP_001165096.1, NP_001165094.1, NP_001028928.1, NP_001020540.2, NP_001020538.2, and NP_001020537.2. Other members of the VEGF family include VEGF-C and VEGF-D.

Disclosed herein are methods for treating pyogenic granuloma by administering an anti-VEGF agent to a subject. In one embodiment, the anti-VEGF antibody bevacizumab can be used in the disclosed methods. The antibody bevacizumab and its VEGF-binding activity are reviewed in detail in Ferrara et al., Biochem. Biophys. Res. Commun., 2005, 333, 328-335. Bevacizumab may be administered to skin or the eye (e.g., for the treatment of pyogenic granulomas) at doses determined by a physician. In one embodiment, the dose for administration to site of the pyogenic granuloma (e.g., for treatment of the granuloma lesion) is about 1.25 mg.

It is to be understood, however, that the treatment method described herein can also be performed using other anti-VEGF agents (e.g., VEGF or VEGFR inhibitors, such as, but not limited to, other anti-VEGF antibodies, drugs, prodrugs, small molecules, peptides, nucleic acid inhibitors (e.g., siRNA, shRNA, antisense oligonucleotides), fusion proteins, etc.), either that are known in the art or that will be discovered or engineered in the future, so long as the anti-VEGF agent has the ability to inhibit the action of VEGF (e.g., human VEGF) and/or a VEGFR (e.g., VEGFR-1, VEGFR-2, and/or VEGFR-3) (e.g., human VEGFR-1, human VEGFR-2, or human VEGFR-3) (i.e., to inhibit VEGF signaling). VEGF and VEGFR inhibitors are discussed in detail in Masabumi Shibuya, Genes & Cancer, 2(12), 1097-1105 (2011), which is incorporated herein by reference.

By way of non-limiting example, other anti-VEGF antibodies and inhibitors that are known in the art, and, that can be used in the methods disclosed herein include but are not limited to: ranibizumab, pegaptanib, imatinib, vandetanib, sorafenib, pazopanib, valatanib, vevasiranib, aflibercept, etanercept, anecortave acetate (angiostatic steroid), VEGF-trap (a fusion protein), squalamine lactate, erlotinib, gefitinib (small molecules), Combretastatin A4 Prodrug (an antitubulin/antiangiogenic agent), AdPEDF (Adenovector pigment epithelium-derived factor), Cand5 (siRNA), protein tyrosine kinase 7 inhibitors (PTK7), lipolytic agents, TG100841, AG013958, AL39324, AGN211745 (VEGF receptor blockers), anti-angiogenic VEGF-A(xxx)b family, VEGF Trap (receptor decoy), protein kinase antibodies to tyrosine kinase inhibitor receptors SIM010603, kinase domain receptor antibodies (KDR1.3 and KDR2.6), GS101 aganirsen (an antisense oligonucleotide against insulin receptor substrate aka IRS-1), picropodophyllin (PPP), tetrameric tripeptide, tissue kallikrein, KH906 (a recombinant human VEGF receptor protein fusion), beta-adreno receptor blocker .beta.3-AR, nicotinic acetycholine receptor antagonists, linomide analogue (Lin05), morpholino oligomers (VEGFR1_MOe13), decursin, prorenin, vasohibin and sirolimus. It will be appreciated that because the amino acids sequences (as well as nucleic acid sequences encoding the amino acid sequences) of VEGF and VEGFRs are known in the art, the skilled artisan can readily design additional anti-VEGF agents for use in the presently disclosed methods.

Dosage ranges for anti-VEGF agents, like those disclosed above, can be readily determined by the ordinarily skilled artisan, and can first be determined in animal models for determining dosage, safety and efficacy according to standard methods known in the art.

### Description of Nanoparticles

As used herein, nanoparticles are intended to mean particles generally ranging in cross-section from 1 nm to about 100 microns. The nanoparticles described herein are designed to be used with or carry anti-VEGF compounds. More generally, nanoparticle therapeutics are typically particles comprised of therapeutic entities, such as small-molecule drugs, peptides, proteins and nucleic acids, and components that assemble with the therapeutic entities, such as lipids and polymers, to form nanoparticles.

Nanoparticles are described herein as part of a smart drug delivery system. The Inventors have discovered that nanoparticles possess tremendous potential for therapeutics delivery of the disclosed compositions to areas of abnormal or excessive angiogenesis. These novel delivery systems can enhance efficacy, while simultaneously reducing side effects, owing to properties such as more targeted localization in tumors and active cellular uptake. The nanoparticles that are candidates for use in drug delivery systems can be categorized into two classes: inorganic and organic nanoparticles. In some embodiments, the compositions disclosed herein contain inorganic nanoparticles. In other embodiments, the compositions disclosed herein contain organic nanoparticles. In further embodiments still, the compositions disclosed herein contain both inorganic and organic nanoparticles.

In some embodiments, inorganic nanoparticles may include those that have physicochemical properties that can be used for both diagnostic purpose such as imaging and treatment purpose such as targeted destruction. In certain embodiments, inorganic particles may include semiconductor quantum dots, super parmagnetic iron oxides, and gold- and/or silver-based particles. In specific embodiments, inorganic nanoparticles made of a composition such as silicon may be coated in gold and/or silver.

In some embodiments, organic nanoparticles may include soft types (e.g. paclitaxel-loaded polymeric micelles, liposomal doxorubicin, and paclitaxed-loaded human serum albumin nanoaggregate) and porphysome nanovesicles and conductive polymer nanoparticles.

In some embodiments, the nanoparticles may include combinations of any of the above plus the "all-in-one" type of nanoparticle such as a porphyrin-based organic nanoparticle and a porphyrin/cholic acid hybrid polymer.

In one embodiment, there is described a method of photo thermally triggering delivery of the anti-VEGF compounds described herein using various organic or inorganic nanoparticles.

In one embodiment, the nanoparticles may comprise gold, silver, and/or iron-oxide, which possess certain photothermal properties.

In one embodiment, there is described anti-VEGF compounds comprising gold and silver nanoparticle to mediate inhibition of angiogenesis.

In one embodiment, there is described anti-VEGF compound including gold nanoparticles to photothermally control the release of angiogenesis inhibiting agents with photo or radio wave activation.

In one embodiment, there is described metal nanoparticles, such as gold nanoparticles, conjugated with an anti-angiogenic peptide which can be combined with visible laser irradiation to enhance angiogenesis arrest *in vivo.* The combination of a green laser coupled to gold nanoparticles can achieve high localized temperatures able to precisely cauterize blood vessels, that when combined with VEGF pathway inhibition, such as the transdermal application of anti-VEGF, can reduce FLT-1 expression.

In one embodiment, there is described a method of photothermally triggering delivery with laser light. In one non-limiting embodiment, a 532 nm laser may be used in conjunction with gold nanoparticles that have been conjugated with anti-angiogenic peptides. It has been discovered that the laser causes a significantly higher increase in temperature for long term low intensity exposure. This has the effect of increasing activity and at the same time coagulating vessels. In another embodiment, there is described RF or light interaction with other nanoparticles described herein. This has been shown to enhance activity by heating, or to release conjugated anti-angiogenesis compounds from nanoparticles with thermal shock or pulsed energy.

### Description of Anti-Inflammatory Steroid

Anti-inflammatory steroids are steroidal compounds that have anti-inflammatory activity and include corticosteroids, including glucocorticoids. Glucocorticoids bind to glucocorticoid receptors in the cytoplasm which may increase the transcription of genes coding for anti-inflammatory proteins, including lipocortin-1, interleukin-10, interleukin-1 receptor antagonist and neutral endopeptidase. Glucocorticoids also inhibit the expression of multiple inflammatory genes, including genes for various cytokines, enzymes, receptors and adhesion molecules. *See, e.g.,* Barnes et al., Clin Sci, 1998, 94, 557-572, which is incorporated herein by reference.

Anti-inflammatory steroids described herein may be used in a variety of forms including (1) creams, which are a mixture of water and oils and usually contains a preservative; (2) ointments, which are made of oils and little to no water and don't usually contain a preservative; (3) gels, which are made with water and propylene glycol; and (4) solutions, foams, and lotions, which typically contain oil and water, and other chemicals.

In some embodiments, the compositions described herein may include any known anti-inflammatory steroid. Non-limiting examples of such steroids include triamcinolone acetonide (TAC) suspension which is typically administered by injection typically in concentrations ranging from 2 mg/ml to 40 mg/ml, prednisolone acetate 1% and/or difluprednate 0.05% (for eye), or alclometasone 0.005% cream, ointment, gel or solution for each one

Other anti-inflammatory steroids that may be used in the described composition include Class IV, moderately potent topical steroids, in cream, ointment, gel or lotion form. Non-limiting examples of such steroids include: flurandrenolide 0.05%, triamcinolone acetonide 0.1%, mometasone furoate 0.1%, fluticasone propionate 0.05%, triamcinolone acetonide 0.1 or 0.025%, clobetasol proprionate 0.05%, diflorasone 0.005% cream, fluocinonide cream 0.1%, and/or prednicarbate topical 0.1 % cream or ointment (for skin), and an NSAID (e.g., diclofenac 1% gel, diclofenac 1.5% solution (topical)).

In general, triamcinolone acetonide and triamcinolone diacetate are the most widely used intralesional corticosteroids, although dexamethasone, betamethasone, or methylprednisolone acetate are used by some clinicians. Triamcinolone agents are available as micronized suspensions. Characteristics associated with micronized suspensions that make them desirable for intralesional administration are the small size of the corticosteroid particles, stability at room temperature, and ease of resuspension of corticosteroid particles with gentle mixing. Small corticosteroid crystals are more efficiently delivered to the treatment site, thereby decreasing the total administered dose of the drug and reducing the risk of systemic side effects and skin atrophy. In addition, because micronized crystals of corticosteroid are in a depot form, the active ingredients are stored in the tissues and released over a period of weeks, making this type of corticosteroid delivery system well suited for the treatment of chronic inflammatory dermatoses. Examples of chronic inflammatory dermatoses that are particularly amenable to this type of extended action include psoriasis, lichen simplex chronicus, cutaneous lupus erythematosus, and prurigo nodularis. In preferred embodiments, the anti-inflammatory steroid may be alclometason, diflorasone, fluocinonide, triamcinolone, prednicarbate, or prednisolone.

### Description of Non-Steroidal Anti-inflammatory Drugs (NSAIDs)

Non-steroidal anti-inflammatory drugs (NSAIDs) are non-steroidal compounds that reduce inflammation. Most NSAIDs act as nonselective inhibitors of the enzyme cyclooxygenase (COX), inhibiting both the cyclooxygenase-1 (COX-1) and cyclooxygenase-2 (COX-2) isoenzymes. COX catalyzes the formation of prostaglandins. Since COX-1 inhibition is believed to be associated with gastrointestinal side-effects of NSAIDs, compounds that are selective COX-2 inhibitors have also been developed.

In some embodiments, the compositions described herein may include any known NSAID In preferred embodiments, the NSAID may be diclofenac.

### Methods of Treatment

In some embodiments, a form of treating excessive or abnormal angiogenesis includes the application of an anti-VEGF agent either independently or in conjunction with at least one or more anti-inflammatory steroid drugs or NSAIDs to the affected patient's skin or eye.

In some embodiments, the anti-VEGF agent is combined with at least one organic, inorganic, or hybrid nanoparticle to form a composition that can be injected into or around the area containing the abnormal or excessive angiogenesis. In certain embodiments, this composition can be injected before, after, or before and after the application of one or more anti-inflammatory steroids or NSAIDs.

In one embodiment, the anti-inflammatory steroids or NSAIDs can also be applied via an injection into or around the area containing the abnormal or excessive angiogenesis. In another embodiment, the anti-inflammatory steroid or NSAID can be applied via the use of eyedrops or any other delivery mechanism known in the art.

In certain embodiments, where the area of treatment is the eye, eyelid, and orbital lesions, the area where the excessive or abnormal angiogenesis is formed can selected from the following: Cavernous hemangiomas, Cavernous malformations, Arteriovenous malformations, Arteriovenous fistulas, Aneurysms, Lymphangiomas, Hemangiopericytomas, Malignant hemangioendotheliomas (angiosarcomas), Angiolymphoid hyperplasias with eosinophilia (Kimura's disease), Racemose hemangiomas of Wybum-Mason syndrome, Hemangioblastomas, Orbital varices, Venous lymphatic malformations, Choroidal hemangiomas, Choroidal melanomas, and Coats Disease.

### Carrier

As mentioned, the composition described herein comprises at least one anti-vascular endothelial grown factor (anti-VEGF) antibody and a carrier, such as nanoparticles. The carrier may comprise extracelluar matrix components, such as collagens, elastic fiber components, hyaluronic acid, glycosaminoglycans, proteoglycans including decorin and versican, PMMA (poly-methyl-methacrylate beads, triamcinolone acetonide suspension (TAC/Kenalog-typically in 10 or 40 mg/ml), albumin including human recombinant or animal albumin, mitomycin C, beta blockers, triamcinolone acetonide (TAC), or other carrier proteins or steroids capable of slow release known to those of ordinary skill in the art.

### Delivery Device

In some embodiments, a delivery device for administering the disclosed composition can be configured to administer the composition by at least one method selected from: intravenous, subconjunctival, subtenon, episcleral, intrascleral, subscleral, intraperitoneal, epidural, intrathecal, intramuscular, intraluminal, intratracheal, epidermal, intradermal, subdermal or subcutaneous.

In one embodiment, the drug delivery device comprises a microneedle array. For example, such an array comprises a patch of multiple tiny needles that delivers the described composition into the skin. This delivery system is particularly useful when the area of excessive or abnormal angiogenesis is on the skin. In this embodiment, the patch may be applied like a bandage, such as a Band-Aid^{®}. In this embodiment, the needles, which are made entirely of sugar and the protein pieces, simply dissolve into the skin.

In another embodiment, where the disorder to be treated is in the periorbital and/or orbit area of an eye, such as pyogenic granuloma, the delivery system may comprise a therapeutic bandage lens, such as a bandage contact lens (BCL). These types of therapeutic lenses are defined as any contact lens used to promote healing, relieve pain and protect the ocular surface, including scleral contact lenses. Scleral contact lenses, which instead of covering only a portion of the cornea (like conventional lenses), are larger and cover the entire corneal surface and rest on the "white" of the eye (the sclera). The placement of these therapeutic lenses, whether temporary or as part of a long-term treatment plan, are part of a medical treatment or procedure to deliver the compositions described herein to a pyogenic granuloma. Therefore, in one embodiment, the VEGF antibody containing compositions described herein are applied as a coating on the BCL to provide a long term, and direct release of the disclosed composition to the location of the pyogenic granuloma.

In another embodiment, the drug delivery device comprises punctal plugs, which are defined as tiny medical devices that sit in the tear ducts in the eyes to help prevent tears draining away. In one embodiment, scleral contact lens and/or punctal plugs, coupled with anti VEGFs and nanoparticles, are devices that can be placed in the periocular areas and periorbital areas near the eye to treat pyogenic granuloma on the globe and other tumors mentioned in this application.

In some embodiments, a delivery device for administering the disclosed composition can be configured to administer the composition and carrier into the affecting tissue at high speeds. For example, the composition and carrier may be injected at supersonic speeds to subsurface tissues. In these embodiments, the delivery device may include a computer-controlled application station, an applicator, and treatment cartridges. In certain embodiments, the computer-controlled application station can be operated via touchscreen, allowing a user to select a program depending on the indication and active ingredient. Without being bound to theory, it is believed that the preset programs ensure standardized and efficient operation of the application system.

In some embodiments, the delivery device can be a part of a kit consisting of at least one syringe, vial or applicator preloaded with a predetermined amount of the pharmaceutical composition, as described in more detail below.

### Kits

In certain embodiments, the present disclosure provides kits for treating excessive or abnormal angiogenesis in a subject. The kits can include an anti-VEGF antibody (e.g., bevacizumab, Ranibizumab, Pegaptanib) or anti-VEGF agent (e.g., Anecortave acetate, angiostatic steroid), as well as a means of administration including a patch composed of the agents in crystallized tiny needle form applied by a band-aid, a single of multi-needle injector or any other device for administering a compound sub-epidermally.

The kit may further include one or more of an anti-inflammatory steroid described herein. The skilled artisan will appreciate that the dosages of the above anti-inflammatory steroids and NSAIDs may be varied without departing from the nature of the present disclosure, and thus other dosages are also encompassed by the present disclosure. The skilled artisan will know which dosages of the anti-inflammatory steroids and NSAIDs disclosed herein may be safely and effectively administered to a subject according to the standard of care and knowledge in the art and can include the anti-inflammatory steroids and NSAIDs described herein.

The kit may further include a smart drug delivery systems described herein. In particular, the kit may include inorganic or organic nanoparticles dispersed in a suitable carrier, such as a cream or gel, that can encapsulate and deliver the described active ingredients to the location of treatment. In one embodiment, the carrier comprises extracelluar matrix components, such as collagens, elastic fiber components, hyaluronic acid, glycosaminoglycans, proteoglycans including decorin and versican, PMMA (poly-methyl-methacrylate beads, triamcinolone acetonide suspension (TAC/Kenalog-typically in 10 or 40 mg/ml), albumin. Other carriers for nanoparticles are disclosed in Li et al., Nat. Commun., 5, 4712 (2014), which is incorporated herein by reference.

In a specific embodiment, a kit includes bevacizumab; one or more of prednisolone acetate 1% and difluprednate 0.05% and triamcinolone acetonide, and a nanoparticle carrier.

In another specific embodiment, a kit includes at least one sterile therapeutic bandage lens for the eye. In this embodiment, the therapeutic bandage lens has located on the surface a composition comprising one or more of: an anti-VEGF antibody (e.g., bevacizumab, Ranibizumab, Pegaptanib) or anti-VEGF agent (e.g., Anecortave acetate, angiostatic steroid); an anti-inflammatory steroid described herein; a NSAIDs described herein, all of which may be included in a carrier, such as one comprising extracelluar matrix components, such as collagens, elastic fiber components, hyaluronic acid, glycosaminoglycans, proteoglycans including decorin and versican, triamcinolone acetonide suspension, and albumin.

In certain embodiments, the kit is for treating excessive or abnormal angiogenesis in a subject, such as a pyogenic granuloma. The kit can further optionally include instructions for use.

In one embodiment, the kit may include syringes or applicators preloaded with the above mentioned agents and/or vials containing one or more of the agents.

### Measurement/Examination Techniques

*Slit Lamp Examination:* Slit lamp examination involves the use of a device with a low-powered microscope and a slit lamp to conduct an examination on a patient's eye. The slit lamp emits a high-intensity light that allows for a closer examination of the eye. Eyedrops containing a dye or dilation drops may be used to allow for a more precise examination of the eye. The slit lamp has different filters to get different views of the eye and the device can be used to examine various parts of the eye including the eyelids, conjunctiva, iris, lens, sclera, cornea, retina, and optic nerve.

In some embodiments, known forms of examination are used to identify the location of lesions in the body. These examination techniques include: Optical Coherent Tomography, Positron Emission Tomography, Dual Modal Positron Emission Tomography - Magnetic Resonance Imaging, Magnetic Resonance Imaging, or a combination of the disclosed method.

The efficacy of therapeutics for certain treatments, such as tumors is hampered by multiple barriers to drug delivery, including severe destabilizing effects in the blood circulation, the blood-brain barrier/blood-brain tumor barrier (BBBIBB'ΓB), and limited tumor uptake. Accordingly, in one embodiment there is disclosed a Sequential Targeting In Crosslinking (STICK) nanodelivery system to overcome these important physiological barriers and to improve drug delivery to certain lesions located in or on the body, including excessive or abnormal angiogenesis in a subject, such as a pyogenic granuloma or various tumors. In one embodiment, contrast agents, which include a STICK nanodelivery system, with lipophobic, lipophilic and pH sensitive properties, are used to penetrate blood brain barriers, blood tumor barriers and other organ or tissue barriers. In certain embodiments, the disclosed nanoparticles may be coupled with an anti-VEGF contrasting agent to enhance the visibility of any lesions and activate the anti-VEGF agent. In these embodiments, nanoparticles may be introduced into the body in two application. In the first application, the nanoparticles paired with the contrasting agent may be used to identify any lesions. In the second application, the nanoparticles may be introduced to the site of the lesion carrying a protein that would change the configurations of the receptions. This allows the anti-VEGF agent to go to the cells.

### Anti-Tumor Agents and Nanoparticulate Delivery Systems

In various embodiments, the compositions described herein may include at least one anti-tumor agent, such as Mitomycin or fluorouracil (5 FU). Fluorouracil is an anticancer ("antineoplastic" or "cytotoxic") chemotherapy drug and is classified as an "antimetabolite." These agents can be used to prevent the formation of pyogenic granuloma. Therefore, in one embodiment, the composition can be used for specialized chemo and/or chemo-radiation therapy that delivers a high dose of anti-tumor agent directly to the tumor to kill more tumor cells and cause less damage to normal tissue. These drugs that are used in chemotherapy, such as fluorouracil and Mitomycin C, work in different ways to stop the growth of tumor cells, either by killing the cells or by stopping them from dividing.

As one skilled in the art would appreciate, anti-tumor agents can be highly toxic and have unwanted and often dangerous side effects if the dosages are not precisely given. As described above, an additional benefit of the nanoparticulate delivery systems disclosed herein is that they allow for a precise delivery of the described therapeutics to the exact location of treatment, which mitigate or eliminate toxic side effects to surrounding tissue.

### Devices such as jet-injectors, needles including arrays of microneedle injectors, and atomized sprayers

A device configured to deliver the AVEGF-containing composition and compounds thereof into target tissue at predetermined depths. Lower concentrations of A VEGF can be used if applied directly to the area of interest rather than migration through a concentration gradient in tissue. In one embodiment AVEGF is applied by microneedles over a predetermine area and over a predetermined range of depths where the area predominantly matches the area of the target tissue and the range of depths extends mostly through the entire depth of the target tissue whereby the target tissue is an area subject to scar or keloid formation.

A device with multiple needles spaced such that anti-VEGF solution is dispersed completely into the target area such as an area predisposed to developing keloids, or a surgical scar.

In one embodiment, the following description is meant to be used by injection, multi-needle injectors, or jet power injector. Methods and devices are used to ensure a slow, sustained or timed release of anti-VEGF compounds, as described below:
a) combining them with fillers such as saline, hyaluronic acid (HA), collagen, elastin, proteoglycans such as decorin, versican, chondroitin sulfate, herparin sulphate proteoglycan, elastin, fibrillin, fibulin (refer to abandon filler patent attached), collagens (all types), and the like;
b) combining with albumin and similar carrier proteins,
c) liposomes and liposomes that can be triggered by light, lasers, ultrasound, or decay over time;
d) use of colloidal or polymeric capsule for micro-encapsulation and nanoencapsulation of AVEGF solutions for controlled release of AVEGF and injectors for administering micro-encapsulated A VEGF solutions;
e) Compounds including AVEGF with corticosteroids, hydroquinone and other drugs.
f) In combination with stem cell therapy, growth factors, and PRP specifically to prevent stem cell stimulated angiogenesis.

Methods of injecting with compounded with saline, sterile water, one of the above listed or a combination. A device of a pre-filled cartridge for injection of one of the described compounds is also disclosed. In one embodiment, the concentration of AVEGF containing compound, such as bevacizumab, used in the disclosed and inventive embodiments, range from 0.025 to 250 mg/ml, such as 1.0 to 100 mg/ml, 10 to 50 mg/ml, 20 to 30 mg/ml, such as 25 mg/ml. These endpoints may be used in any combination such as 30 to 100 mg/m l.

In one embodiment, there is described a pre-filled device, such as a cartridge, syringe, vial, applicator, patch or other delivery device comprising bevacizumab in an amount of 2.5 mg/0.1 mL (25 mg/mL).

Accordingly, in various aspects of the present disclosure, there is described a nanoparticulate delivery system that achieves one or more of the following benefits: highly specific drug targeting and delivery; reduced toxicity while maintaining therapeutic effects; greater safety and biocompatibility; and faster delivery of the compositions described herein to the treatment area.

### EXAMPLE - TREATMENT OF PYOGENIC GRANULOMA WITH BEVACIZUMAB WITH COMBINATION THERAPY

The following non-limiting example, which are intended to be exemplary, further clarify the present disclosure.

### TREATMENT PROTOCOL

A topical anesthetic (proparacaine 0.5% eye drops or Alcaine manufactured by Alcon Laboratories, Inc.) was applied to the eye following recent pterygiectomy and amniotic membrane graft surgery. The patients were examined to identify any abnormal grown on the eye. If an abnormal growth was found in the examination, the patient was treated with a combination of bevacizumab (Avastin) (Genentech/Roche), an anti-inflammatory steroid drug such as triamcinolone (Kenalog) (Bristol-Myers Squibb), and/or an NSAIDs such ketorolac injection (Toradol) (Roche) or ketorolac eye drops (Acular) (Allergan).

A syringe containing approximately 0.05 mL of bevacizumab (1.25 mL/0.05 mL) was injected into the eye and the plunger was drawn back to determine if blood was present in the syringe hub. If blood was present (indicating presence of a blood vessel), the syringe needle was withdrawn, and the plunger was drawn back again until no blood was observed in the syringe hub. Bevacizumab was then injected into the pyogenic granuloma lesion and/or in areas adjacent to the lesion where blood vessels were dilated and numerous. If bleeding occurred (e.g., in the form of subconjunctival or subcutaneous hematoma), tamponade with Q-tips was performed until bleeding stopped; then injection was resumed.

The anti-inflammatory steroid drug and/or NSAID were administered either before or after the bevacizumab injection. The anti-inflammatory steroid drug and/or NSAID were administered by injection and/or via eye drops If the anti-inflammatory steroid drug and/or NSAID was administered by injection, it was done following a similar process as described above for the bevacizumab injection. A syringe containing approximately 0.03 mL triamcinolone injection (40 mg/mL) was injected into the pyogenic granuloma lesion and the plunger was drawn back to determine if blood was present in the syringe hub. If blood was present (indicating presence of a blood vessel), the syringe needle was withdrawn, and the procedure repeated until no blood was observed in the syringe hub. The anti-inflammatory steroid drug or NSAID was injected into the pyogenic granuloma lesion and/or in areas adjacent to the lesion where blood vessels were dilated and numerous. If bleeding occurred *(e.g.,* in the form of subconjunctival or subcutaneous hematoma), tamponade with Q-tips was performed until bleeding stopped; then injection was resumed. If the anti-inflammatory steroid drug or NSAID was delivered by eyedrops, the steroid eye drops were given for approximately 6 weeks 1 to 4 times a day (every 6 hours) or once every hour (24 drops/day). NSAID eye drops were given 4 times a day every 6 hours for 3 to 6 months.

Patients were followed up with to monitor for appearance of new pyogenic granuloma. The presence of new pyogenic granuloma was an indication for further treatment (e.g. with injection and/or eye drops). In some cases, during the first surgery to remove pterygium, the pterygium was only partially removed (e.g. to prevent necrosis). Thus, in some patients, additional surgeries were subsequently performed to remove remaining sections of the pterygium, resulting in additional pyogenic granuloma.

Two patients (Patients 1 and 2) underwent surgery to remove primary pterygium (pterygiectomy) from the cornea of one or both eyes followed by amniotic membrane graft surgery and were treated as described below.

### PATIENT 1 - RIGHT EYE

An 88-year old patient presented with occasional ocular tearing bilaterally without any pain or discomfort. Slit lamp examination revealed a 4.2 mm nasal pterygium in the right eye. The patient underwent a combined pterygiectomy and amniotic membrane graft surgery and was doing well postoperatively with no complications. At his 60-day postoperative follow up, a new growing, red, and raised lesion, identified as a pyogenic granuloma, was found in his right eye.

The following drug treatments were given on the number of days indicated after his pterygiectomy and amniotic graft surgery:
Day 90: 0.03 mL triamcinolone injection (40 mg/mL) to the lesion.
Day 97: 0.05 mL bevacizumab injection (1.25 mg/mL) to the lesion.

On day 104 after the patient's surgery, the pyogenic granuloma was considerably smaller in circumference and showed no evidence of hyperemia. The pyogenic granuloma completely resolved 30 days later.

The above results demonstrated that pyogenic granuloma lesions were inhibited when the patient received a combination therapy including administration of bevacizumab and an anti-inflammatory steroid (injected)

### PATIENT 2 - LEFT EYE

A 77-year-old male presented with a 6-month history of bilateral pterygia, 4.6 mm in the right eye and 4.8 mm in the left eye. He had undergone multiple pterygiectomy and amniotic membrane grafts in both eyes for recurrent pterygium. The patient did well post-operatively and the graft was present in the right eye for 3 months. Months after completing a series of pyerygiectomy, the patient's left eye developed a pyogenic granuloma.

The following drug treatments were given 98 days after his pterygiectomy and amniotic graft surgery:
Day 98: 0.03 mL triamcinolone injection (40 mg/mL) to the lesion.
Day 100: 0.05 mL bevacizumab injection (1.25 mg/mL) to the lesion

Significant progress was shown within two weeks of the bevacizumab injection. Although the pyogenic granuloma was still present in the left eye, its size was significantly smaller, and it was less erythematous. At the patient's 1-month follow-up visit, the pyogenic granuloma completely resolved and there was no evidence of recurrence.

The above results demonstrated that pyogenic granuloma lesions were inhibited when the patient received a combination therapy including administration of bevacizumab and an anti-inflammatory steroid (injected or topical).

### PATIENT 3 - RIGHT EYE

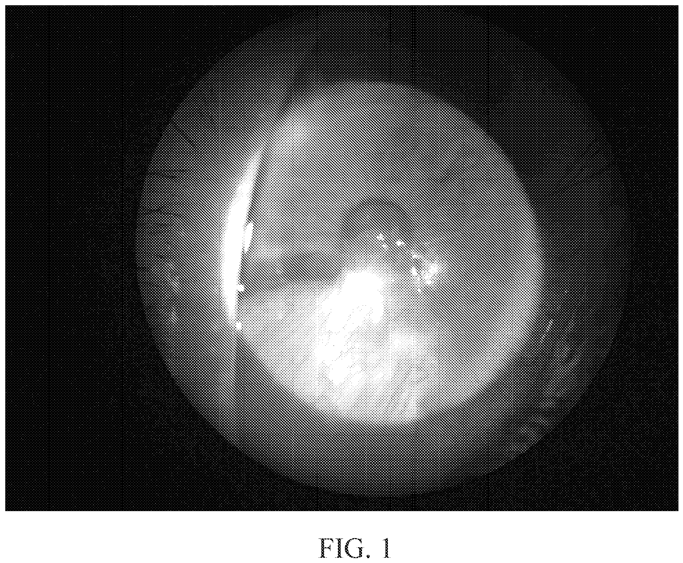

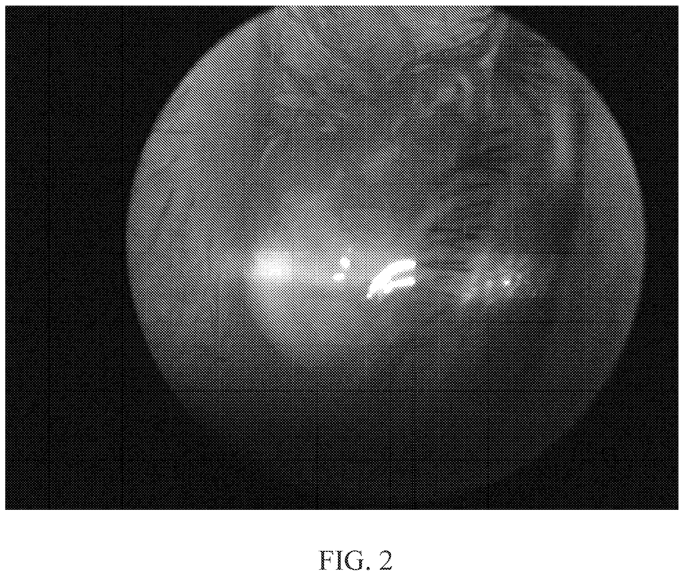

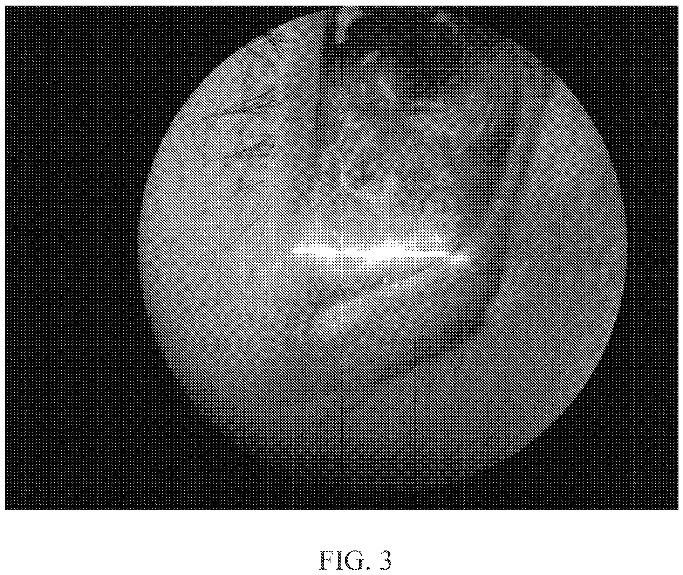

A 55-year old male with pterygium in the right eye measured 6.4 mm. Figure 1 shows the patient's eye prior to receiving pterygiectomy and amniotic membrane graft. The patient developed a pyogenic granuloma in the right eye following the pterygiectomy and amniotic membrane graft, as shown in Figure 2, and 60 days after the pterygiectomy and amniotic membrane graft he received the following treatments:
Day 60: 003 mL triamcinolone injection (40 mg/mL) to the lesion. Figure 3 shows the patient's eye following injection of triamcinolone.
Day 68: 005 mL bevacizumab injection (1.25 mg/mL) to the lesion.

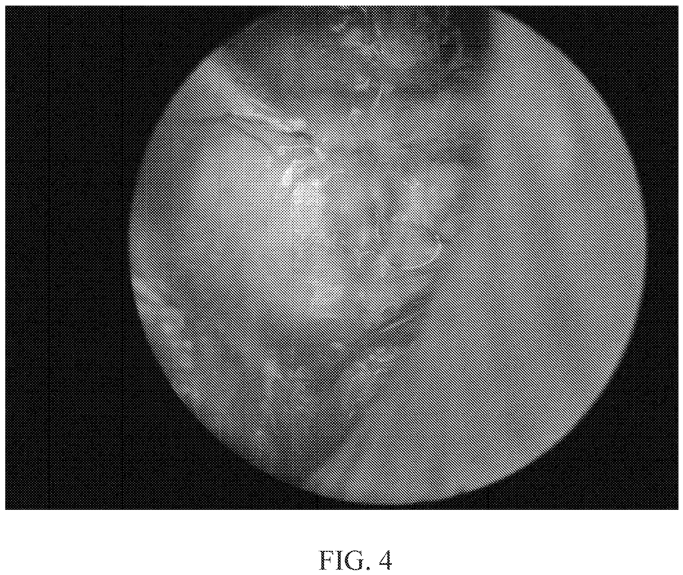

On day 86 the pyogenic granuloma in the right eye completely resolved, as shown in Figure 4.

The above results demonstrated that pyogenic granuloma was eliminated when the patient received a combination therapy including administration of bevacizumab, an anti-inflammatory steroid (injected or topical), and a topical NSAID, and that the combination therapy was superior to treatment with bevacizumab alone.

Other embodiments of the invention will be apparent to those skilled in the art from consideration of the specification and practice of the invention disclosed herein. It is intended that the specification and examples be considered as exemplary only, with the true scope of the invention being indicated by the following claims.

### Comprising the following clauses:

1. A composition for the treatment abnormal or excessive angiogenesis, comprising:
   at least one anti-vascular endothelial grown factor (anti-VEGF) antibody; and
   a carrier comprising nanoparticles,
   wherein the nanoparticles are organic, inorganic, or a combination thereof.
2. The composition of clause 1, wherein the anti-VEGF antibody comprises bevacizumab, ranibizumab, lapatinib, sunitinib, sorafenib, axitinib, pazopanib, or combination thereof.
3. The composition of clause 2, wherein the anti-VEGF antibody is bevacizumab.
4. The composition of clause 3, wherein the concentration of bevacizumab in a solution for administration is 1.25 mg/0.05 mL.
5. The composition of clause 1, wherein the nanoparticles are organic.
6. The composition of clause 5, wherein the organic nanoparticles
   comprise
   porphysome nanovesicles, or conductive polymer nanoparticles.
7. The composition of clause 6, wherein the organic nanoparticles comprise at least one of paclitaxel-loaded polymeric micelles, liposomal doxorubicin, or paclitaxel-loaded human serum albumin nanoaggregate.
8. The composition of clause 1, wherein the nanoparticles are inorganic.
9. The composition of clause 8, wherein the inorganic nanoparticles
   comprise
   semiconductor quantum dots, super paramagnetic iron oxide, and gold-based compounds.
10. The composition of clause 1, wherein the nanoparticles area combination of porphyrin-based organic nanoparticles and the porphyrin/cholic acid hybrid polymers.
11. The composition of clause 1, further comprising an anti-inflammatory steroid, a non-steroidal anti-inflammatory drug (NSAID), or both.
12. The composition of clause 11, wherein the anti-inflammatory steroid is alclometason, diflorasone, fluocinonide, triamcinolone, prednicarbate, or prednisolone.
13. The composition of clause 11, wherein the NSAID is diclofenac, ketorolac, neparenac, or bromfenac.
14. The composition of clause 1, wherein the pyogenic granuloma is present on the periorbital and/or orbital areas of the eye.
15. The composition of clause 1, wherein the pyogenic granuloma is present on the skin.
16. The composition of clause 1, wherein the area containing abnormal or excessive angiogenesis comprises pyogenic granuloma, infantile hemangioma, hemangioma of infancy, congenital hemangioma, tufted hemangioma, spindle cell hemangioma, epithelioid hemangioma, kaposiform hemangioendothelioma, retiform hemangioendothelioma, papillary intralymphatic angioendothelioma, dabska tumor, composite hemangioendothelioma, Kaposi sarcoma, angiosarcoma, Epithelioid hemangioendothelioma, capillary malformation, lymphatic malformation, venous malformation, arteriovenous malformation, arteriovenous fistula, capillary-lymphatic malformation, capillary-arteriovenous malformation, capillary-lymphatic-arteriovenous malformation, capillary-venous-arteriovenous malformation, capillary-lymphatic-venous-arteriovenous malformation.
17. The composition of clause 16, wherein the area containing abnormal or excessive angiogenesis comprises at least one eye, eyelid and orbital lesions selected from: Cavernous hemangiomas, Cavernous malformations, Arteriovenous malformations, Arteriovenous
   fistulas, Aneurysms, Lymphangiomas, Hemangiopericytomas, Malignant hemangioendotheliomas (angiosarcomas), Angiolymphoid hyperplasias with eosinophilia (Kimura's disease), Racemose hemangiomas of Wybum-Mason syndrome, Hemangioblastomas, Orbital varices, Venous lymphatic malformations, Choroidal hemangiomas, Choroidal melanomas, and Coats Disease.
18. A method of treating abnormal or excessive angiogenesis, the method comprising:
   administering to an area containing abnormal or excessive angiogenesis a composition containing at least one anti-VEGF antibody and nanoparticles, wherein the nanoparticles are organic, inorganic, or a combination thereof
   administering an anti-inflammatory steroid; and
   administering a non-steroidal, anti-inflammatory drug (NSAID).
19. The method of clause 18, wherein the steps can be performed in any order or simultaneously.
20. The method of clause 18, wherein the anti-VEGF antibody is bevacizumab.
21. The method of clause 20, wherein the bevacizumab is administered at a dosage
   of 1.25 mg.
22. The method of claim 18, wherein the nanoparticles are organic and comprise porphysome nanovesicles, or conductive polymer nanoparticles.
23. The method of clause 18, wherein the organic nanoparticles comprise at least one of paclitaxel-loaded polymeric micelles, liposomal doxorubicin, or paclitaxel-loaded human serum albumin nanoaggregate.
24. The method of clause 18, wherein the nanoparticles are inorganic.
25. The method of clause 24, wherein the inorganic nanoparticles comprise semiconductor quantum dots, super paramagnetic iron oxide, and gold-based compounds.
26. The method of clause 18, wherein the nanoparticles are a combination of porphyrin-based organic nanoparticles and the porphyrin/cholic acid hybrid polymers.
27. The method of clause 18, wherein the composition is administered by injection or topically.
28. The method of clause 18, wherein the composition, the anti-inflammatory steroid, and the NSAID are all administered on the same day or individually on separate days.
29. The method of clause 28, wherein the composition, the anti-inflammatory steroid, and the NSAID are all administered at least once.
30. The method of clause 18, wherein the anti-inflammatory steroid is one or more of alclometason, diflorasone, fluocinonide, triamcinolone, prednicarbate, or prednisolone.
31. The method of clause 30, wherein the anti-inflammatory steroid is triamcinolone.
32. The method of clause 30, wherein the triamcinolone is administered at a dose of 12 mg.
33. The method of clause 18, wherein the NSAID is one or more of diclofenac, ketorolac, neparenac, or bromfenac.
34. The method of clause 18, wherein the area containing abnormal or excessive angiogenesis is present in the eye.
35. The method of clause 18, wherein the area containing abnormal or excessive angiogenesis is present on the skin.
36. The method of clause 18, wherein the area containing abnormal or excessive angiogenesis comprises pyogenic granuloma, infantile hemangioma, hemangioma of infancy, congenital hemangioma, tufted hemangioma, spindle cell hemangioma, epithelioid hemangioma, kaposiform hemangioendothelioma, retiform hemangioendothelioma, papillary intralymphatic angioendothelioma, dabska tumor, composite hemangioendothelioma, Kaposi sarcoma, angiosarcoma, Epithelioid hemangioendothelioma, capillary malformation, lymphatic malformation, venous malformation, arteriovenous malformation, arteriovenous fistula, capillary-lymphatic malformation, capillary-arteriovenous malformation, capillary-lymphatic-arteriovenous malformation, capillary-venous-arteriovenous malformation, capillary-lymphatic-venous-arteriovenous malformation.
37. The method of clause 18, wherein the area containing abnormal or excessive angiogenesis comprises at least one eye, eyelid and orbital lesions selected from: Cavernous hemangiomas, Cavernous malformations, Arteriovenous malformations, Arteriovenous fistulas
   Aneurysms, Lymphangiomas, Hemangiopericytomas, Malignant hemangioendotheliomas (angiosarcomas), Angiolymphoid hyperplasias with eosinophilia (Kimura's disease), Racemose hemangiomas of Wybum-Mason syndrome, Hemangioblastomas, Orbital varices, Venous lymphatic malformations, Choroidal hemangiomas, Choroidal melanomas, and Coats Disease.
38. A device for administering the composition of clause 1, the device configured to administer the composition by at least one method selected from intravenous, subconjunctival, subtenon, episcleral, intrascleral, subscleral, intraperitoneal, epidural, intrathecal, intramuscular, intraluminal, intratracheal, epidermal, intradermal, subdermal or subcutaneous.
39. The device of clause 38, wherein said device comprises one part of a kit.
40. The device of clause 39, wherein the kit comprises at least one cartridge, syringe, vial or applicator preloaded with a predetermined amount of the pharmaceutical composition.

## Claims

1. A composition for the treatment abnormal or excessive angiogenesis of at least one pterygium or pterygium reoccurrence, the composition comprising: at least one anti-vascular endothelial grown factor (anti-VEGF) agent; and a carrier comprising nanoparticles, wherein the nanoparticles are organic.

2. A composition containing at least one anti-VEGF agent and nanoparticles for use in a method of treating abnormal or excessive angiogenesis of a pterygium or pterygium reoccurrence in at least one eye of a subject, the method comprising:
at least one administration of the composition to the at least one eye, wherein
the nanoparticles are organic, and wherein
the composition is administered after the subject has undergone surgery to remove the pterygium.

3. The composition for use according to claim 2, wherein the method further comprises at least one second administration of the composition after the first administration; optionally
wherein the method further comprises at least one third administration and/or surgery to remove the pterygium or pterygium reoccurrence after the at least one second administration.

4. The composition of claim 1, or the composition for use according to claim 2,
wherein the nanoparticles are organic and comprise porphysome nanovesicles, or conductive polymer nanoparticles; optionally
wherein the organic nanoparticles comprise at least one of paclitaxel-loaded polymeric micelles, liposomal doxorubicin, or paclitaxel-loaded human serum albumin nanoaggregate.

5. The composition of claim 1, or the composition for use according to claim 2,
wherein the nanoparticles are organic;
wherein the organic nanoparticles comprise semiconductor quantum dots, super paramagnetic iron oxide, and gold-based compounds.

6. The composition of claim 1, or the composition for use according to claim 2,
wherein the nanoparticles are a combination of porphyrin-based organic nanoparticles and the porphyrin/cholic acid hybrid polymers.

7. The composition for use according to claim 2, wherein the composition is administered by injection.

8. The composition of claim 1, or the composition for use according to claim 2, wherein the composition further comprises an anti-inflammatory steroid, a non-steroidal anti-inflammatory drug (NSAID), or both..

9. The composition of claim 8, or the composition for use according to claim 8, wherein the anti-inflammatory steroid is one or more of alclometason, diflorasone, fluocinonide, triamcinolone, prednicarbate, or prednisolone; optionally, wherein the anti-inflammatory' steroid is triamcinolone; further optionally, wherein the triamcinolone is administered at a close of 12 mg; or
wherein the NSAID is one or more of diclofenac, ketorolac, neparenac, or bromfenac.

10. The composition of claim 1, or the composition for use according to claim 2, wherein the pterygium or pterygium reoccurrence comprises pyogenic granuloma.

11. A device for administering the composition of claim 1, the device configured to administer the composition by at least one method selected from intravenous, subconjunctival, subtenon, episcleral, intrascleral, or subscleral.

12. A kit for treating abnormal or excessive angiogenesis of a pterygium or pterygium granuloma, the kit comprising
a composition comprising an anti-VEGF agent;
one or more carriers comprising nanoparticles; and
a device for administration.

13. The kit of claim 12, wherein the kit comprise a composition according to any one of claims 1, 3 to 6 and 8 to 11.

14. The kit of claim 12 or 13, wherein the device for administration comprises a device according to claim 11.

15. The kit of claim 12 or 13, wherein the kit further comprises:
one or more anti-inflammatory steroids, NSAIDs, or both;
instructions for use; and/or
at least one cartridge, syringe, vial or applicator preloaded with a predetermined amount of the composition.
